(19) Europäisches Patentamt
European Patent Office
Office européen des brevets

(11) Veröffentlichungsnummer: **0 464 639 A1**

# (12) EUROPÄISCHE PATENTANMELDUNG

(21) Anmeldenummer: **91110571.6**

(51) Int. Cl.5: **C07D 405/06**, C07D 317/28

(22) Anmeldetag: **26.06.91**

(30) Priorität: **02.07.90 DE 4021007**

(43) Veröffentlichungstag der Anmeldung:
**08.01.92 Patentblatt 92/02**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Kleeman, Heinz-Werner, Dr.**
**Jahnstrasse 6**
**W-6092 Kelsterbach(DE)**
Erfinder: **Henning, Rainer, Dr.**
**Losbacher Strasse 4 g**
**W-6234 Hatterheim a. M(DE)**
Erfinder: **Urbach, Hansjörg, Dr.**
**Le Lavandoustrasse 41**
**W-6242 Kronberg/Taunus(DE)**

## (54) Aminodol-Derivate.

(57) Die Erfindung betrifft Verbindungen der Formel

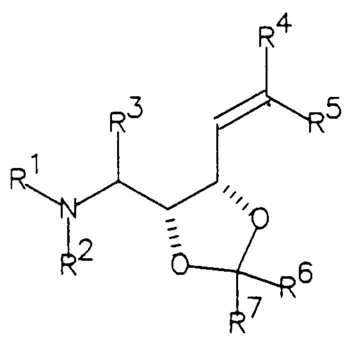

in welcher $R^1$ und $R^2$ gleich oder verschieden sind und Wasserstoff oder Aralkyl bedeuten, $R^3$ Alkyl, Cycloalkyl oder Cycloalkylalkyl bedeutet, $R^4$ und $R^5$ gleich oder verschieden sind und Wasserstoff, Alkyl, Cycloalkyl, Cycloalkylalkyl, Aryl, Aralkyl oder einen Heterocyclen-Rest bedeuten und $R^6$ und $R^7$ gleich oder verschieden sind und Wasserstoff, Alkyl, Cycloalkyl, Cycloalkylalkyl, Aryl oder Aralkyl bedeuten oder $R^6$ und $R^7$ zusammen mit dem sie tragenden Kohlenstoffatom Cycloalkyl bedeuten. Die neuen Verbindung werden durch eine Carbonyl-Olefinierung nach Wittig hergestellt. Sie sind Zwischenprodukte bei der Herstellung von Pharmazeutika, insbesondere von Inhibitoren des Renins und der HIV-Protease.

EP 0 464 639 A1

Die Erfindung betrifft Verbindungen der Formel I

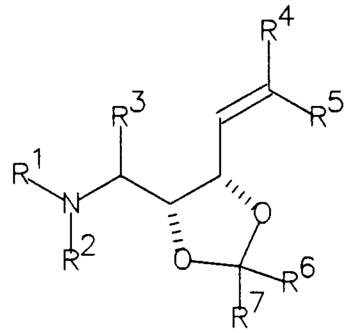

(I)

in welcher
$R^1$ und $R^2$ gleich oder verschieden sind und Wasserstoff, 1-[($C_6$-$C_{14}$)-Aryl]-($C_1$-$C_6$)-alkyl, 1,1-Di-[($C_6$-$C_{14}$)-aryl]-($C_1$-$C_6$)-alkyl oder 1,1,1-Tri-[($C_6$-$C_{14}$)-aryl]-($C_1$-$C_6$)-alkyl bedeuten;
$R^3$ ($C_1$-$C_{12}$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_6$)-alkyl bedeutet, wobei der Cycloalkylteil jeweils gegebenenfalls mit ($C_1$-$C_6$)-Alkyl substituiert ist;
$R^4$ und $R^5$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, wobei Aryl jeweils mit ein, zwei oder drei Resten der Gruppe ($C_1$-$C_6$)-Alkyl, ($C_1$-$C_4$)-Alkoxy oder Halogen substituiert sein kann, Het oder Het-($C_1$-$C_6$)-alkyl bedeuten, wobei Het für einen 5-, 6- oder 7-gliedrigen heterocyclischen Ring steht, der gegebenenfalls benzanelliert ist und aromatisch, teilhydriert oder vollständig hydriert sein kann und der als Heteroelement ein oder zwei gleiche oder verschiedene Reste aus der Gruppe N, O, S, NO, SO und $SO_2$ enthält und der mit ein oder zwei gleichen oder verschiedenen Resten aus der Gruppe ($C_1$-$C_6$)-Alkyl, ($C_1$-$C_4$)-Alkoxy und Halogen substituiert sein kann; und
$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl oder ($C_6$-$C_{14}$)-Aryl-($C_1$-$C_6$)-alkyl bedeuten, wobei Aryl jeweils mit ein, zwei oder drei gleichen oder verschiedenen Resten aus der Gruppe ($C_1$-$C_6$)-Alkyl, ($C_1$-$C_4$)-Alkoxy und Halogen substituiert sein kann; oder $R^6$ und $R^7$ zusammen mit dem sie tragenden Kohlenstoffatom ($C_3$-$C_{12}$)-Cycloalkyl bedeuten;
sowie deren Salze.

Alkyl kann geradkettig oder verzweigt sein. Das gleiche gilt für davon abgeleitete Reste wie Alkoxy.

Unter Cycloalkyl versteht man z.B. Cyclopropyl, Cyclobutyl, Cyclopentyl, Cyclohexyl oder Cycloheptyl.

Ein als Het bezeichneter Rest hat vorzugsweise eine der folgenden Bedeutungen: Pyridyl, Thiazolyl, Thienyl, Pyranyl, Benzofuryl, Isobenzofuryl, Furyl, Pyrrolyl, Imidazolyl, Pyrazinyl, Indazolyl, Chinolyl, Isochinolyl, Phthalazinyl, Pyrimidinyl, Pyrazinyl, Indolizinyl, Isoindolyl, Indolyl, Chinoxalinyl, Chinazolinyl, Cinnolinyl, Oxazolyl, Isoxazolyl, Isothiazolyl. Die Reste können aromatisch, teilhydriert oder vollständig hydriert sein. Sie können mit einem oder zwei gleichen oder verschiedenen Resten aus der Gruppe ($C_1$-$C_6$)-Alkyl, ($C_1$-$C_4$)-Alkoxy und Halogen substituiert sein.

Unter ($C_6$-$C_{14}$)-Aryl versteht man beispielsweise Phenyl, Naphthyl oder Biphenylyl; bevorzugt ist Phenyl.

Halogen ist Fluor, Chlor, Brom oder Iod.

Bevorzugt sind Verbindungen der Formel I, in welcher
$R^1$ und $R^2$ gleich oder verschieden und Wasserstoff, 1-[($C_6$-$C_{14}$)-Aryl]-($C_1$-$C_6$)-alkyl, 1,1-Di-[($C_6$-$C_{14}$)-aryl]-($C_1$-$C_6$)-alkyl bedeuten;
$R^3$ ($C_1$-$C_6$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_3$)-alkyl bedeutet;
$R^4$ und $R^5$ wie oben definiert sind; und
$R^6$ und $R^7$ gleich oder verschieden sind und Wasserstoff; ($C_1$-$C_{12}$)-Alkyl, ($C_3$-$C_{12}$)-Cycloalkyl, ($C_3$-$C_{12}$)-Cycloalkyl-($C_1$-$C_6$)-alkyl bedeuten; oder diese zusammen mit dem sie tragenden Kohlenstoffatom für ($C_3$-$C_{12}$)-Cycloalkyl stehen;
sowie deren Salze.

Besonders bevorzugt sind Verbindungen der Formel I, in welcher $R^1$ und $R^2$ gleich oder verschieden und Wasserstoff, Benzyl, 1-Phenylethyl oder Diphenylmethyl bedeuten;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;
$R^4$ und $R^5$ wie oben definiert sind; und
$R^6$ und $R^7$ gleich sind und Wasserstoff oder ($C_1$-$C_4$)-Alkyl bedeuten, oder diese zusammen mit dem sie tragenden Kohlenstoffatom für ($C_5$-$C_{17}$)-Cycloalkyl stehen;
sowie deren Salze.

Ganz besonders bevorzugt sind Verbindungen der Formel I, in welcher
$R^1$ und $R^2$ gleich oder verschieden sind und Wasserstoff, Benzyl, 1-Phenylethyl oder Diphenylmethyl bedeuten;
$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;
$R^4$ und $R^5$ gleich oder verschieden sind und Wasserstoff, ($C_1$-$C_4$)-Alkyl, $C_5$-$C_7$)-Cycloalkyl, ($C_5$-$C_7$)-Cycloalkyl-($C_1$-$C_3$)-alkyl, Phenyl, Benzyl, 2-Pyridyl, 3-Pyridyl, 4-Pyridyl, 2-Thiazolyl, 4-Thiazolyl, 5-Thiazolyl, 2-Thienyl, 3-Thienyl, 1-Methylimidazol-2-yl, 1-Methylimidazol-4-yl oder 1-Methylimidazol-5-yl bedeuten; und
$R^6$ und $R^7$ gleich sind und Wasserstoff, ($C_1$-$C_4$)-Alkyl, oder zusammen mit dem sie tragenden Kohlenstoffatom ein ($C_5$-$C_7$)-Cycloalkyl bedeuten;
sowie deren Salze.

Die Erfindung betrifft weiterhin ein Verfahren zur Herstellung von Verbindungen der Formel I, das dadurch gekennzeichnet ist, daß man eine Verbindung der Formel IV

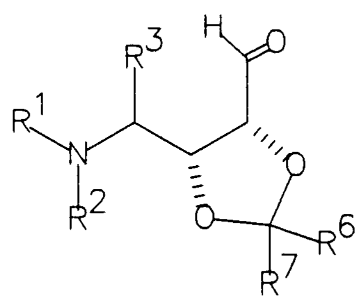

(IV)

in welcher die Reste $R^1$, $R^2$, $R^3$, $R^6$ und $R^7$ wie oben definiert sind, umsetzt mit einem Phosphoran der Formel V, in welcher $R^4$ und $R^5$ wie oben definiert sind und $R^8$, $R^9$ und $R^{10}$ für gleiche oder verschiedene Arylreste, vorzugsweise Phenyl steht.

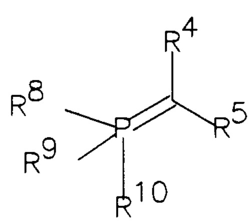

(V)

Zur Herstellung einer Verbindung der Formel IV kann man von geeignet geschützten Furanose-N-Glycosiden wie D-Mannofuranose-N-Glycosiden, L-Gulofuranose-N-Glycosiden, D-Talofuranose-N-Glycosiden oder L-Allofuranose-N-Glycosiden ausgehen. Die Umsetzung mit einem C-Nucleophil wie einer Grignard-Verbindung oder einer Alkyllithium-Verbindung in einem gegen diese Nucleophile inerten Lösungsmittel wie Diethylether, Di-n-butylether, MTB, DIP, THF, Tetrahydropyran, Formaldehyddimethylacetal oder DME bei einer Temperatur zwischen -30 °C und dem Siedepunkt des Lösungsmittels, bevorzugt zwischen -10 °C und +35 °C, gegebenenfalls mit Ultraschallunterstützung, liefert das Derivat der Formel II, in welcher die Reste $R^1$, $R^2$, $R^3$, $R^6$ und $R^7$ wie oben definiert sind.

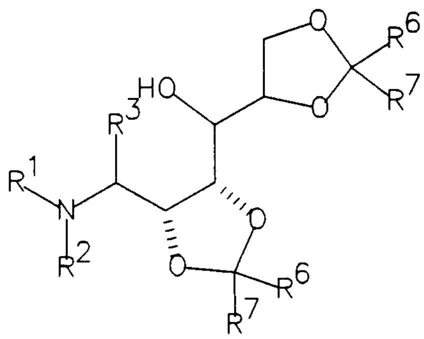

**(II)**

Die Umsetzung mit Säuren in geeigneten Lösungsmitteln wie Wasser, Methanol oder Ethanol bei einer Temperatur von 0 °C bis 65 °C, bevorzugt bei 0 °C bis 30 °C, liefert Derivate der Formel III, in welcher die Reste $R^1$, $R^2$, $R^3$, $R^6$ und $R^7$ wie oben definiert sind.

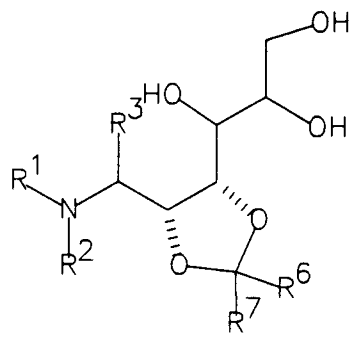

**(III)**

Als Säuren kommen Carbonsäuren wie Essigsäure oder Sulfonsäuren wie p-Toluolsulfonsäure in Frage.

Die Umsetzung mit 1,2-Diole spaltenden Oxidationsmitteln wie $NaIO_4$ oder $Pb(OAc)_4$, $Ca(OCl)_2$, $Bu_4NIO_4$, $MnO_2$, $Tl^{3+}$, $Co^{3+}/O_2$ oder $H_5IO_6$ in inerten Lösungsmitteln wie Wasser, Diethylether, Di-n-butylether, MTB, DME, DIP, THF oder Dioxan bei 0 °C bis 50 °C, bevorzugt bei 0 °C bis 30 °C liefert den Aldehyd der Formel IV, in welcher die Reste $R^1$, $R^2$, $R^3$, $R^6$ und $R^7$ wie oben definiert sind.

Die Wittig-Reaktion mit einem geeigneten Phosphoran in einem inerten Lösungsmittel wie Diethylether, Di-n-butylether, MTB, DIP, THF, DME, Dioxan bei -30 °C bis zum Siedepunkt des Lösungsmittels, bevorzugt zwischen 0 °C und 30 °C liefert die Titelverbindungen der Formel I. Ebenso kann man den Aldehyd der Formel IV mit dem Anion eines geeigneten Phosphinoxids unter Bildung der Titelverbindung umsetzen (Horner; siehe Krauch, Kunz, Reaktionen der Organischen Chemie, Hüthig Verlag Heidelberg 1976, Seite 241).

In einigen Kombinationen der Reste $R^1$ - $R^7$ sind solche $R^1$ und $R^2$, die bei der Synthese der Derivate der Formel II günstig sind, bei der Synthese der Derivate der Formel IV ungünstig. In solchen Fällen wird zweckmäßigerweise eine Umschützung vorgenommen:

Das Derivat der Formel III wird hierzu entweder einer Hydrierung mit Wasserstoff und Pd/C oder einer Transferhydrierung zum Beispiel mit Ammoniumformiat oder Cyclohexen und Pd/C unterworfen. Geeignete Lösungsmittel sind Alkohole wie Methanol und Ethanol. Die Temperatur wird zweckmäßigerweise zwischen -20 °C und dem Siedepunkt des Lösungsmittels gewählt, bevorzugt sind 0 °C bis zum Siedepunkt.

Das Rohprodukt der Hydrierung wird nun mit geeigneten, die neuen $R^1$ und $R^2$ tragenden Elektrophilen umgesetzt in einem Lösungsmittel wie Diethylether, Tetrahydrofuran oder t-Butanol mit einer Hilfsbase wie Triethylamin, Ethyldiisopropylamin oder N-Methylpiperidin. Geeignete Elektrophile sind zum Beispiel Chloride, Bromide, Iodide, Methylsulfonate, Toluolsulfonate oder Trifluormethansulfonate. Zur Erhöhung der Reaktionsgeschwindigkeit bei Verwendung von Chloriden oder Bromiden kann zweckmäßigerweise wasserfreies Natriumiodid zugesetzt werden. Die Umsetzungstemperatur wird zwischen 0 °C und dem Siedepunkt des Lösungsmittels, bevorzugt 20 °C und dem Siedepunkt gewählt.

Die erfindungsgemäßen Verbindungen der Formel I sind wertvolle Zwischenprodukte bei der Herstellung von Pharmazeutika, insbesondere von Inhibitoren des Renins und der HIV-Protease. Inhibitoren, bei deren Synthese die Verbindungen der Formel I vorteilhaft verwendet werden können, sind zum Beispiel in FEBS Lett. 230, 38(1988), J. Med. Chem. 31, 2264 (1988), J. Med. Chem. 31, 2277 (1988), Biochem.

Biophys. Res. Commun. 146 959 (1987) und EP-A-370 454 beschrieben.

Verzeichnis der verwendeten Abkürzungen:

| | |
|---|---|
| DC | Dünnschichtchromatographie |
| DCI | Desorption Chemical Ionisation |
| DIP | Diisopropylether |
| DME | 1,2-Dimethoxyethan |
| EE | Essigsäureethylester |
| FAB | Fast atom bombardment |
| Hep | n-Heptan |
| M | Molekularpeak |
| MeOH | Methanol |
| MS | Massenspektrum |
| MTB | Methyl-tert.-butylether |
| NEM | N-Ethylmorpholin |
| R.T. | Raumtemperatur |
| Schmp. | Schmelzpunkt |
| THF | Tetrahydrofuran |

Die nachstehenden Beispiele dienen zur Erläuterung der vorliegenden Erfindung, ohne daß diese darauf beschränkt wäre:

Beispiel 1

N,N-Dibenzyl-[1-Cyclohexylmethyl-(2,3-isopropyliden)-2(R),3(S)-dihydroxy-5-(2-pyridyl)-pent-4-en-1-yl]amin

1,3 g N-N-Dibenzyl-[1-Cyclohexylmethyl,(2,3-isopropyliden)-2(R),3(R),4(R),5(R),6-pentahydroxy]-hexylamin wird in 65 ml Diethylether gelöst und 25 ml 1 M $KH_2PO_4$-Lösung zugegeben. Dann wird beim Raumtemperatur 2,7 g $NaIO_4$ in 50 ml $H_2O$ zugegeben und bei dieser Temperatur 4 Tage gerührt. Anschließend wird mit 50 ml 10 % wäßrigen $Na_2SO_3$-Lösung versetzt, 3 x mit 100 ml Diethylether extrahiert, die organische Phase über $Na_2SO_4$ getrocknet und das Solvens i. Vak. entfernt. Schließlich wird noch 3 h bei Raumtemperatur im Ölpumpenvakuum getrocknet und man erhält den Rohaldehyd.

Nun wird 1,1 g 2-Picolyltriphenylphosphoniumchlorid in 15 ml THF (wasserfrei) suspendiert und 0,3 g Kalium-t-butylat bei Raumtemperatur addiert. Nach 3,5 h wird auf 0 °C gekühlt und obiger Rohaldehyd in 10 ml THF zugetropft. 18 h wird bei Raumtemperatur gerührt, das Reaktionsgemisch in 100 ml gesättigte wäßrige $NaHCO_3$-Lösung gegossen und 3 x mit 100 ml EE extrahiert, über $Na_2SO_4$ wird getrocknet und das Solvens i.Vak. entfernt. Chromatographie an Kieselgel mit Diisopropylether lieferte 980 mg der Titelverbindung als farbloses Öl, laut NMR nahezu ausschließlich trans.

$R_f$ (DIP) = 0,31    MS (DCI) : 511 (M+1)

Beispiel 1a

N,N-Dibenzyl-[1-Cyclohexylmethyl,(2,3-isopropyliden)-2(R),3(R),4(R),5(R)-6-pentahydroxy]-hexylamin

5,2 g N-Benzhydryl-[1-Cyclohexylmethyl-(2,3-isopropyliden)-2(R),3(R),4(R),5(R),6-pentahydroxy]-hexylamin werden in 200 ml Methanol gelöst, 6,8 g Ammoniumformiat sowie 1,0 g 10 % Pd/C addiert und 3 Stunden bei Raumtemperatur gerührt. Der Katalysator wird abfiltriert, das Methanol i.Vak. entfernt, in 200 ml EE/200 ml ges. wäßriger $Na_2CO_3$-Lösung aufgenommen und weitere 2 x mit 200 ml EE extrahiert. Über $Na_2SO_4$ wird getrocknet und das Solvens i.Vak. entfernt. In 100 ml THF (wasserfrei) wird aufgenommen, 2,6 ml Benzylbromid, 3,7 ml Diisopropylethylamin sowie 6,4 g NaI (wasserfrei) zugegeben und 16 Stunden unter Rückfluß erhitzt. Anschließend wird in 250 ml ges. wäßriger $NaHCO_3$ gegossen und 3 x mit 150 ml EE extrahiert. Man trocknet über $Na_2SO_2$, dann wird das Solvens i.Vak. entfernt. Chromatographie an Kieselgel mit MTB liefert 4,2 g der Titelverbindung als weißer Schaum.

$R_f$ (MTB) = 0,42    MS (DCI) : 498 (M+1)

Beispiel 1b

N-Benzhydryl-[1-Cyclohexylmethyl,(2,3-isopropyliden)-2(R),3(R),4(R),5(R),6-pentahydroxy]-hexylamin

1,7 g N-Benzhydryl-[1-Cyclohexylmethyl,(2,3-,5,6-di-isopropyliden)-2(R),3(R),4(R),5(R),6-pentahydroxy]-hexylamin werden in 70 ml Methanol gelöst und mit 1,7 g p-Toluolsulfonsäure versetzt. Man rührt 5 h bei Raumtemperatur, dann wird das Gemisch in 300 ml ges. wäßriger $Na_2CO_3$-Lösung eingetragen, das Methanol i.Vak. entfernt und 3 x mit 150 ml EE extrahiert. Über $Na_2SO_4$ wird getrocknet, das Solvens i.Vak. entfernt und an Kieselgel mit EE chromatographiert. Man erhält 1,2 g der Titelverbindung als weißen Schaum.

$R_f$ (EE) = 0,41 MS (FAB) : 484 (M + 1)

Beispiel 1c

N-Benzhydryl-[1-Cyclohexylmethyl,(2,3-5,6-diisopropyliden)-2(R),3(R),4(R),5(R),6-pentahydroxy]hexylamin

520 mg Lithium-Draht (1 % Na, ∅ 3,2 mm) wird in 12 ml Diethylether (wasserfrei) unter Argon vorgelegt und etwa 40 µl Cyclohexylmethylbromid bei Raumtemperatur zugespritzt und gerührt, bis die Reaktion anspringt, was sich durch Trübung äußert. Dann wird auf - 10 °C gekühlt und weitere 4,2 ml Cyclohexylmethylbromid in 6 ml Diethylether zugetropft. Bei - 10 °C bis 0 °C wird 6 h gerührt, anschließend auf -30 °C gekühlt und auf - 30 °C gekühltes Benzhydrylamino-D-Mannofuranosid-diacetanid in 20 ml Diethylether schnell zugegeben (exotherm!). Nach 15 min ist die Reaktion im DC bereits beendet. Nach 1 h Rühren bei Raumtemperatur werden die Lithium-Reste abfiltriert, die Reaktionslösung in 150 ml ges. wäßriger $NaHCO_3$-Lösung eingetragen und 3 x mit 100 ml Essigester extrahiert. Über $Na_2SO_4$ wird getrocknet, das Solvens i.Vak. entfernt und an 500 g Kieselgel mit Diisopropylether/Toluol 1:5 chromatographiert.

Man erhält 4,6 g der Titelverbindung als farbloses Öl

$R_f$ (DIP) = 0,31 MS (DCI) : 511 (M + 1)

Beispiel 1d

Benzylhydrylamino-Mannofuranosid-diacetonid

10 g D( + )-Mannose und etwa 10 mg p-Toluolsulfonsäure werden in 40 ml 2,2-Dimethoxypropan suspendiert und bei 40 °C 1 h gerührt. Dabei entsteht eine klare Lösung. 10 ml Benzhydrylamin wird zugegeben und 24 h am Rückfluß gekocht. Dann werden weitere 10 ml 2,2-Dimethoxypropan und 2 ml Benzhydrylamin zugegeben und weitere 18 h am Rückfluß gekocht. Flüchtige Bestandteile werden i.Vak. entfernt, in 100 ml EE aufgenommen und dreimal mit 100 ml $NaHCO_3$-Lösung gewaschen. Über $Na_2SO_4$ wird getrocknet, das Solvens i.Vak. entfernt und an Kieselgel mit DIP/Toluol 1:5 chromatographiert. Man erhält 17 g der Titelverbindung als blaßgelbe Kristalle, Fp: 82-84 °C.

$R_f$ (DIP/Toluol 1:3) = 0,41 MS (DCI) : 426 (M + 1)

Alternativsynthese der Titelverbindung des Beispiels 1c:

Beispiel 1e

N-Benzhydryl-[1-Cyclohexylmethyl,(2,3-,5,6-diisopropyliden)-2(R),3(R),4(R),5(R),6-pentahydroxy]hexylamin

1,8 g Benzhydrylamino-Mannofuranosid-diacetonid und 1,2 ml Cyclohexylmethylbromid werden in 40 ml Formaldehyddimethylacetal (von K/Na-Legierung destilliert) gelöst und mit 115 mg Lithiumdraht (3,2 mm, ca. 1 % Na) unter Argon im Ultraschallbad bei 20 - 40 °C 3,5 h umgesetzt. Dann wird erneut 115 mg Lithiumdraht und 1,2 ml Cyclohexylmethylbromid zugesetzt und noch 1 h bei 40 - 60 °C umgesetzt. Man gießt das Reaktionsgemisch in 200 ml $NaHCO_3$-Lösung und extrahiert dreimal mit 100 ml MTB. Über $Na_2SO_4$ wird getrocknet, das Solvens i.Vak. entfernt und an Kieselgel mit DIP/Toluol 1:3 chromatographiert. Man erhält 1,1 g der Titelverbindung als farbloses Öl.

Beispiel 2

N,N-Dibenzyl-[1-Cyclohexylmethyl-(2,3-isopropyliden)-2(R),3(S)-dihydroxy-5-methyl-hex-4-en-1-yl]amin

Die Titelverbindung des Beispiels 2 wurde analog zu Beispiel 1 synthetisiert.

$R_f$ (Dip/Hep 1:10) = 0,33 MS (DCI) : 462 (M + 1)

**Patentansprüche**

**1.** Verbindung der Formel I

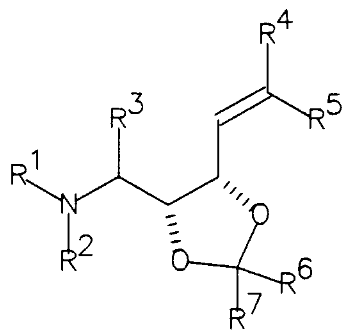

(I)

in welcher

$R^1$ und $R^2$ gleich oder verschieden sind und Wasserstoff, 1-[($C_6$-$C_{14}$)-Aryl]-($C_1$-$C_6$)-alkyl, 1,1-Di-[($C_6$-$C_{14}$)-aryl]-($C_1$-$C_6$)-alkyl oder 1,1,1-Tri-[($C_6$-$C_{14}$)-aryl]-($C_1$-$C_6$)-alkyl bedeuten;

$R^3$ ($C_1$-$C_{12}$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_6$)-alkyl bedeutet, wobei der Cycloalkylteil jeweils gegebenenfalls mit ($C_1$-$C_6$)-Alkyl substituiert ist;

$R^4$ und $R^5$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, wobei Aryl jeweils mit ein, zwei oder drei Resten der Gruppe ($C_1$-$C_6$)-Alkyl, ($C_1$-$C_4$)-Alkoxy oder Halogen substituiert sein kann, Het oder Het-($C_1$-$C_6$)-alkyl bedeuten, wobei Het für einen 5-, 6- oder 7-gliedrigen heterocyclischen Ring steht, der gegebenenfalls benzanelliert ist und aromatisch, teilhydriert oder vollständig hydriert sein kann und der als Heteroelement ein oder zwei gleiche oder verschiedene Reste aus der Gruppe N, O, S, NO, SO und $SO_2$ enthält und der mit ein oder zwei gleichen oder verschiedenen Resten aus der Gruppe ($C_1$-$C_6$)-Alkyl, ($C_1$-$C_4$)-Alkoxy und Halogen substituiert sein kann; und

$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl oder ($C_6$-$C_{14}$)-Aryl-($C_1$-$C_6$)-alkyl bedeuten, wobei Aryl jeweils mit ein, zwei oder drei gleichen oder verschiedenen Resten aus der Gruppe ($C_1$-$C_6$)-Alkyl, ($C_1$-$C_4$)-Alkoxy und Halogen substituiert sein kann; oder $R^6$ und $R^7$ zusammen mit dem sie tragenden Kohlenstoffatom ($C_3$-$C_{12}$)-Cycloalkyl bedeuten;

sowie deren Salze.

**2.** Verbindung der Formel I gemäß Anspruch 1, in welcher

$R^1$ und $R^2$ gleich oder verschieden und Wasserstoff, 1-[($C_6$-$C_{14}$)-Aryl]-($C_1$-$C_6$)-alkyl, 1,1-Di-[($C_6$-$C_{14}$)-aryl]-($C_1$-$C_6$)-alkyl bedeuten;

$R^3$ ($C_1$-$C_6$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_3$)-alkyl bedeutet;

$R^4$ und $R^5$ wie im Anspruch 1 definiert sind; und

$R^6$ und $R^7$ gleich oder verschieden sind und Wasserstoff; ($C_1$-$C_{12}$)-Alkyl, ($C_3$-$C_{12}$)-Cycloalkyl, ($C_3$-$C_{12}$)-Cycloalkyl-($C_1$-$C_6$)-alkyl bedeuten; oder diese zusammen mit dem sie tragenden Kohlenstoffatom für ($C_3$-$C_{12}$)-Cycloalkyl stehen;

sowie deren Salze.

**3.** Verbindung der Formel I gemäß Anspruch 1 oder 2, in welcher

$R^1$ und $R^2$ gleich oder verschieden und Wasserstoff, Benzyl, 1-Phenylethyl oder Diphenylmethyl bedeuten;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;

$R^4$ und $R^5$ wie im Anspruch 1 definiert sind; und

$R^6$ und $R^7$ gleich sind und Wasserstoff oder $(C_1-C_4)$-Alkyl bedeuten, oder diese zusammen mit dem sie tragenden Kohlenstoffatom für $(C_5-C_{17})$-Cycloalkyl stehen;

sowie deren Salze.

**4.** Verbindung der Formel I gemäß einem der Ansprüche 1 - 3, in welcher

$R^1$ und $R^2$ gleich oder verschieden sind und Wasserstoff, Benzyl, 1-Phenylethyl oder Diphenylmethyl bedeuten;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;

$R^4$ und $R^5$ gleich oder verschieden sind und Wasserstoff, $(C_1-C_4)$-Alkyl, $C_5-C_7)$-Cycloalkyl, $(C_5-C_7)$-Cycloalkyl-$(C_1-C_3)$-alkyl, Phenyl, Benzyl, 2-Pyridyl, 3-Pyridyl, 4-Pyridyl, 2-Thiazolyl, 4-Thiazolyl, 5-Thiazolyl, 2-Thienyl, 3-Thienyl, 1-Methylimidazol-2-yl, 1-Methylimidazol-4-yl oder 1-Methylimidazol-5-yl bedeuten; und

$R^6$ und $R^7$ gleich sind und Wasserstoff, $(C_1-C_4)$-Alkyl, oder zusammen mit dem sie tragenden Kohlenstoffatom ein $(C_5-C_7)$-Cycloalkyl bedeuten;

sowie deren Salze.

**5.** Verfahren zur Herstellung von Verbindungen der Formel I gemäß einem der Ansprüche 1 - 4, dadurch gekennzeichnet, daß man eine Verbindung der Formel IV

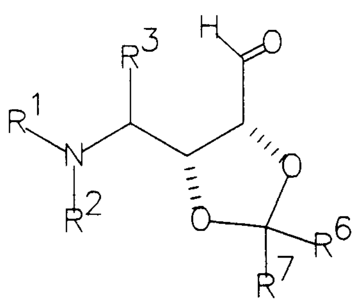

(IV)

in welcher die Reste $R^1$, $R^2$, $R^3$, $R^6$ und $R^7$ wie oben definiert sind, umsetzt mit einem geeigneten Phosphoran.

**6.** Verwendung einer Verbindung gemäß einem der Ansprüche 1 - 4 bei der Herstellung von Pharmazeutika, insbesondere von Inhibitoren des Renins und der HIV-Protease.

**Patentansprüche für folgende Vertragsstaaten: ES, GR**

**1.** Verfahren zur Herstellung von Verbindungen der Formel I

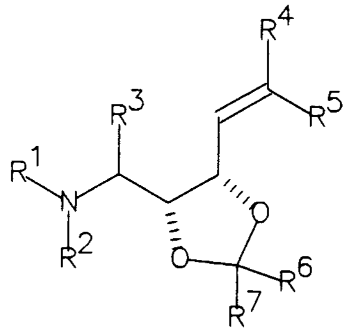

(I)

in welcher

$R^1$ und $R^2$ gleich oder verschieden sind und Wasserstoff, 1-[($C_6$-$C_{14}$)-Aryl]-($C_1$-$C_6$)-alkyl, 1,1-Di-[($C_6$-$C_{14}$)-aryl]-($C_1$-$C_6$)-alkyl oder 1,1,1-Tri-[($C_6$-$C_{14}$-aryl]-($C_1$-$C_6$)-alkyl, wobei Aryl jeweils mit einem mit einem oder zwei gleichen oder verschiedenen Resten aus der Reihe ($C_1$-$C_6$)Alkyl, ($C_1$-$C_4$)Alkoxy und Halogen substituiert sein kann bedeuten;

$R^3$ ($C_1$-$C_{12}$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_6$)-alkyl bedeutet, wobei der Cycloalkylteil jeweils gegebenenfalls mit ($C_1$-$C_6$)-Alkyl substituiert ist;

$R^4$ und $R^5$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, wobei Aryl jeweils mit ein, zwei oder drei Resten der Gruppe ($C_1$-$C_6$)-Alkyl, ($C_1$-$C_4$)-Alkoxy oder Halogen substituiert sein kann, Het oder Het-($C_1$-$C_6$)-alkyl bedeuten, wobei Het für einen 5-, 6- oder 7-gliedrigen heterocyclischen Ring steht, der gegebenenfalls benzanelliert ist und aromatisch, teilhydriert oder vollständig hydriert sein kann und der als Heteroelement ein oder zwei gleiche oder verschiedene Reste aus der Gruppe N, O, S, NO, SO und $SO_2$ enthält und der mit ein oder zwei gleichen oder verschiedenen Resten aus der Gruppe ($C_1$-$C_6$)-Alkyl, ($C_1$-$C_4$)-Alkoxy und Halogen substituiert sein kann; und

$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl oder ($C_6$-$C_{14}$)-Aryl-($C_1$-$C_6$)-alkyl bedeuten, wobei Aryl jeweils mit ein, zwei oder drei gleichen oder verschiedenen Resten aus der Gruppe ($C_1$-$C_6$)-Alkyl, ($C_1$-$C_4$)-Alkoxy und Halogen substituiert sein kann; oder $R^6$ und $R^7$ zusammen mit dem sie tragenden Kohlenstoffatom ($C_3$-$C_{12}$)-Cycloalkyl bedeuten,

sowie deren Salze,

wobei man eine Verbindung der Formel IV

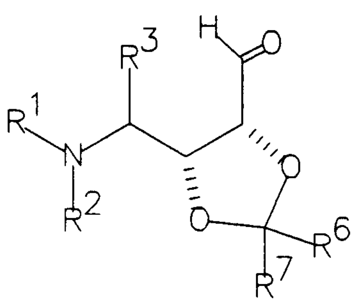

(IV)

in welcher die Reste $R^1$, $R^2$, $R^3$, $R^6$ und $R^7$ wie oben definiert sind, umsetzt mit einem Phosphoran, und die entstehende Verbindung gegebenenfalls in ihre Salze überführt.

**2.** Verfahren gemäß Anspruch 1, dadurch gekennzeichnet, daß die Symbole in Formel (I) folgende Bedeutung haben:

$R^1$ und $R^2$ gleich oder verschieden und Wasserstoff, 1-[($C_6$-$C_{14}$)-Aryl]-($C_1$-$C_6$)-alkyl, 1,1-Di-[($C_6$-$C_{14}$)-aryl]-($C_1$-$C_6$)-alkyl, wobei Aryl jeweils mit einem oder zwei gleichen oder verschiedenen Resten aus der Reihe ($C_1$-$C_6$)Alkyl, ($C_1$-$C_4$)-Alkoxy und Halogen substituiert sein kann;

$R^3$ ($C_1$-$C_6$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_3$)-alkyl;

$R^4$ und $R^5$ wie im Anspruch 1 definiert; und

$R^6$ und $R^7$ gleich oder verschieden sind und Wasserstoff; ($C_1$-$C_{12}$)-Alkyl, ($C_3$-$C_{12}$)-Cycloalkyl, ($C_3$-$C_{12}$)-Cycloalkyl-($C_1$-$C_6$)-alkyl; oder diese zusammen mit dem sie tragenden Kohlenstoffatom ($C_3$-$C_{12}$)-Cycloalkyl.

**3.** Verfahren gemäß Anspruch 1 dadurch gekennzeichnet, daß die Symbole in Formel (I) folgende Bedeutungen haben:

$R^1$ und $R^2$ gleich oder verschieden und Wasserstoff, Benzyl, 2-, 3- oder 4-Methylbenzyl, 2-, 3- oder 4-Methoxybenzyl, 2-, 3- oder 4-Chlorbenzyl, 1-Phenylethyl oder Diphenylmethyl;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl;

$R^4$ und $R^5$ wie im Anspruch 1 definiert; und

$R^6$ und $R^7$ gleich sind und Wasserstoff oder ($C_1$-$C_4$)-Alkyl bedeuten, oder diese zusammen mit dem sie tragenden Kohlenstoffatom ($C_5$-$C_{17}$)-Cycloalkyl.

**4.** Verfahren gemäß Anspruch 1, dadurch gekennzeichnet, daß die Symbole in Formel (I) folgende Bedeutungen haben:

$R^1$ und $R^2$ gleich oder verschieden Wasserstoff, Benzyl, 2-, 3- oder 4-Methylbenzyl, 2-2-, 3- oder 4-Methoxybenzyl, 1-Phenylethyl oder Diphenylmethyl;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl;

$R^4$ und $R^5$ gleich oder verschieden Wasserstoff, ($C_1$-$C_4$)-Alkyl,($C_5$-$C_7$)-Cycloalkyl, ($C_5$-$C_7$)-Cycloalkyl-($C_1$-$C_3$)-alkyl, Phenyl, Benzyl, 2-Pyridyl, 3-Pyridyl, 4-Pyridyl, 2-Thiazolyl, 4-Thiazolyl, 5-Thiazolyl, 2-Thienyl, 3-Thienyl, 1-Methylimidazol-2-yl, 1-Methylimidazol-4-yl oder 1-Methylimidazol-5-yl; und

$R^6$ und $R^7$ gleich Wasserstoff, ($C_1$-$C_4$)-Alkyl, oder zusammen mit dem sie tragenden Kohlenstoffatom ($C_5$-$C_7$)-Cycloalkyl;

**5.** Verwendung einer Verbindung hergestellt gemäß Anspruch 1 bei der Herstellung von Pharmazeutika, insbesondere von Inhibitoren des Renins und der HIV-Protease.

Europäisches Patentamt

# EUROPÄISCHER RECHERCHENBERICHT

Nummer der Anmeldung

EP 91110571.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| D,A | EP - A2 - 0 370 454 (HOECHST) * Ansprüche 1,4 * | 1,6 | C 07 D 405/06<br>C 07 D 317/28 |
| A | CHEMICAL ABSTRACTS, Band 109, Nr. 13, 26. September 1988, Columbus, Ohio, USA ITO, HIROSHI et al. "Preparation of 2-keto-3-deoxyhepto- and octoaldonic acid derivatives as intermediates for drugs and agrochemicals" Seite 669, Spalte 2, Zusammenfassung-Nr. 110 855j & Jpn. Kokai Tokkyo Koho JP 62,258,342 (87,258,342) | 1,6 | |
| A | CHEMICAL ABSTRACTS, Band 111, Nr. 23, 4. Dezember 1989, Columbus, Ohio, USA WILLIAMS, D. R. et al. "Intramolecular cyclizations from N-alkoxyamines. Formation of dialkylsubstituted pyrrolidines and piperidines" Seite 577, Spalte 1, Zusammenfassung-Nr. 214 356e & Tetrahedron Lett. 1989, 30(11), 1327-30 | 1 | RECHERCHIERTE SACHGEBIETE (Int. Cl.⁵)<br>C 07 D 405/00<br>C 07 D 317/00 |
| P,A | CHEMICAL ABSTRACTS, Band 113, Nr. 3, 16. Juli 1990, Columbus, Ohio, USA KIM, YOUNG GYU et al. "Divergent syntheses of stereoisomers of swainsonine: (-)-8-epi, (-)-8a-epi- and (-)-8,8a-diepi- | 1 | |

Der vorliegende Recherchenbericht wurde für alle Patentansprüche erstellt.

| Recherchenort | Abschlußdatum der Recherche | Prüfer |
|---|---|---|
| WIEN | 24-09-1991 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 91110571.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| | -swainsonine" Seite 700, Spalte 1, Zusammenfassung-Nr. 24 297e & Tetrahedron Lett. 1989, 30(42), 5721-4 -- | | |
| P,A | CHEMICAL ABSTRACTS, Band 113, Nr. 25, 17. Dezember 1990, Columbus, Ohio, USA BERGMAN, R. et al. "A total synthesis of (+)-isovelleral. The absolute configuration of the Russulaceae sesquiterpenes" Seite 754, Spalte 1, Zusammenfassung-Nr. 231 702y & J. Chem. Soc., Chem. commun. 1990, (12), 865-7 ---- | 1 | |

RECHERCHIERTE SACHGEBIETE (Int. Cl.⁵)

Der vorliegende Recherchenbericht wurde für alle Patentansprüche erstellt.

| Recherchenort | Abschlußdatum der Recherche | Prüfer |
|---|---|---|
| WIEN | 24-09-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument